# FASCICULE DE BREVET EUROPEEN

(11) **EP 0 399 867 B1**
(45) Date de publication et mention de la délivrance du brevet: **20.07.1994**
(21) Numéro de dépôt: 90401225.9
(22) Date de dépôt: 09.05.1990
(51) Int. Cl.: C07C 19/055, C07C 17/00

(54) **Procédé de synthèse du 1,1,1,2-tétrachloroéthane par hydrochloration du trichloréthylène**
Verfahren zur Herstellung von 1,1,1,2-Tetrachlorethan durch Hydrochlorierung von Trichlorethylen
Process for the preparation of 1,1,1,2-tetrachloroethane by hydrochlorination of trichloroethylene

(30) Priorité: 26.05.1989 FR 8906923
(43) Date de publication de la demande: 28.11.1990
(73) Titulaire: ELF ATOCHEM S.A., 92800 Puteaux (FR)
(72) Inventeur: Commandeur, Raymond, F-38220 Vizille (FR); Correia, Yves, F-04160 Chateaux-Arnoux (FR)

(56) Documents cités:
- FR-A- 977 398

## Description

La présente invention concerne un procédé de synthèse du 1,1,1,2-tétrachloréthane par hydrochloration du trichloréthylène.

Le 1,1,1,2-tétrachloréthane (CCl₃CH₂Cl) est un produit utile comme solvant et surtout comme intermédiaire de synthèse organique. Le brevet allemand DE 1 964 551 (Chemical Abstracts 76641 n, vol. 73) décrit la chloration d'un mélange de 1,1,1-trichloréthane (C Cl₃CH₃) et de trichloréthylène (CHCl=CCl₂) pour obtenir un mélange de 1,2-dichloréthane (CH₂ClCH₂Cl), pentachloréthane et 1,1,1,2-tétrachloréthane. Le brevet allemand DE 1 817 193 (Chemical Abstracts 123515 v, vol. 71) décrit la chloration d'un mélange de 1,1-dichloréthylène (CCl₂=CH₂) et de 1,2-dichloréthylène (CHCl=CHCl) pour produire un mélange de 1,1,1,2-tétrachloréthane et 1,1,2,2-tétrachloréthane (CHCl₂-CHCl₂). Le brevet allemand DE 1 817 194 (Chemical Abstracts 123516 w, vol. 71) décrit la chloration du 1,1-dichloréthylène pour produire du 1,1,1,2-tétrachloréthane.

Le brevet US 3 860 666 concerne la préparation du perchloréthylène par chloration catalytique de l'éthylène dans un lit fluide d'alumine. Il se forme aussi un peu de trichloréthylène. Une faible partie de l'alumine est convertie en chlorure d'aluminium et catalyse l'hydrochloration du trichloréthylène en 1,1,1,2-tétrachloréthane. Le trichloréthylène ne représente que quelques pourcent du mélange en sortie du lit fluide et seule une faible partie est convertie en 1,1,1,2-tétrachloréthane. A l'échelle industrielle on ne peut pas utiliser le chlorure d'aluminium pour catalyser l'hydrochloration du trichloréthylène en 1,1,1,2-tétrachloréthane parce que le trichloréthylène est instable en présence de chlorure d'aluminium et on risque une décomposition.

On trouve dans un article de M.S. KARASCH, J.A. NORTON et F.R. MAYO, J. Org. Chem. 3, 1938, pages 48-54 une hydrochloration du trichloréthylène, catalysée par le chlorure ferrique à la température ambiante. Le taux de conversion du trichloréthylène ne dépasse pas 49 % après 6 jours de réaction.

Le brevet US 3 732 322 (Chemical Abstracts 4985 d, vol. 79) décrit la deshydrochloration du 1,1,1,2-tétrachloréthane en présence de FeCl₃ pour produire du trichloréthylène. Malgré cet enseignement montrant soit une hydrochloration, soit une deshydrochloration sous l'effet de FeCl₃, la demanderesse a mis au point un procédé d'hydrochloration du trichloréthylène en présence de FeCl₃ et sous pression. Ce procédé est sélectif en isomère 1,1,1,2-tétrachloréthane et il est réalisable à l'échelle industrielle. La demanderesse a aussi découvert que, hors le chlorure ferrique et le chlorure d'aluminium déjà cités, les halogénures métalliques étaient des catalyseurs d'hydrochloration du trichloréthylène pour produire sélectivement du 1,1,1,2-tétrachloréthane.

La présente invention est donc un procédé d'hydrochloration du trichloréthylène en présence de chlorure ferrique et à une pression supérieure à la pression atmosphérique.

Il est avantageux d'opérer à une pression supérieure à 5 bars absolus, et de préférence comprise entre 10 et 40 bars absolus. On ne sortirait pas du cadre de l'invention en opérant à une pression supérieure, mais les difficultés techniques de mise en oeuvre rendent le procédé compliqué sans autre avantage. La température peut être quelconque mais il est avantageux d'opérer entre 10 et 160°C, et de préférence entre 20 et 100°C.

Selon la température et la pression, les réactifs peuvent être liquides ou gazeux. Il est préférable d'opérer en milieu anhydre. Les réactifs peuvent être dilués dans un solvant pourvu qu'il soit inerte dans les conditions de la réaction.

La quantité de chlorure ferrique est avantageusement comprise entre 0,1 et 20 %, et de préférence entre 1 et 5 % en poids du trichloréthylène.

La réaction de l'invention peut être mise en oeuvre dans tout dispositif assurant une mise en contact du chlorure ferrique du trichloréthylène et de l'acide chlorhydrique. En fin de réaction, on peut éventuellement laver le milieu réactionnel. Le 1,1,1,2-tétrachloréthane est séparé par exemple par distillation.

Les exemples suivants illustrent l'invention. Dans ces exemples, en fin de réaction, le mélange réactionnel est lavé à l'eau puis analysé par chromatographie en phase gazeuse.

### EXEMPLE 1 (non conforme à l'invention)

Dans un réacteur en verre agité, équipé d'un thermomètre, d'un injecteur d'HCl et d'un réfrigérant ascendant, on charge 2 moles de trichloréthylène (263 g), le chlorure ferrique, on porte à 35°C et on introduit 1 mole/heure d'HCl. N'ayant décelé au bout d'une heure aucune réaction, on continue l'injection d'HCl au même débit mais la température du réacteur a été portée à 40°C. Après une heure à 40°C on continue l'injection d'1 mole/heure d'HCl mais la température a été portée à 60°C. Au bout d'une heure (c'est-à-dire 1 heure à 35°C, 1 heure à 40°C et 1 heure à 60°C), on trouve 0,25 % de CCl₃CH₂Cl dans le réacteur. Les résultats sont portés sur le tableau I.

### EXEMPLES 2 ET 3

Dans un réacteur Inox de un litre, on charge 2 moles de trichloréthylène et le catalyseur ; on place sous la pression d'HCl indiqué dans le tableau I à la température ambiante, on ferme l'autoclave et on porte à la température indiquée, pendant le temps indiqué dans le tableau I. La pression maximum obtenue dans l'exemple 2 est 60 bars et 85 bars dans l'exemple 3.

### EXEMPLES 4 ET 5

Dans un réacteur de 4 litres en acier verré et muni d'une agitation, on place :
- 20 moles de trichloréthylène
- le catalyseur

et sous la pression d'HCl indiquée au tableau I on maintient l'autoclave à la température (au tableau I) et pendant le temps indiqué dans le tableau I.

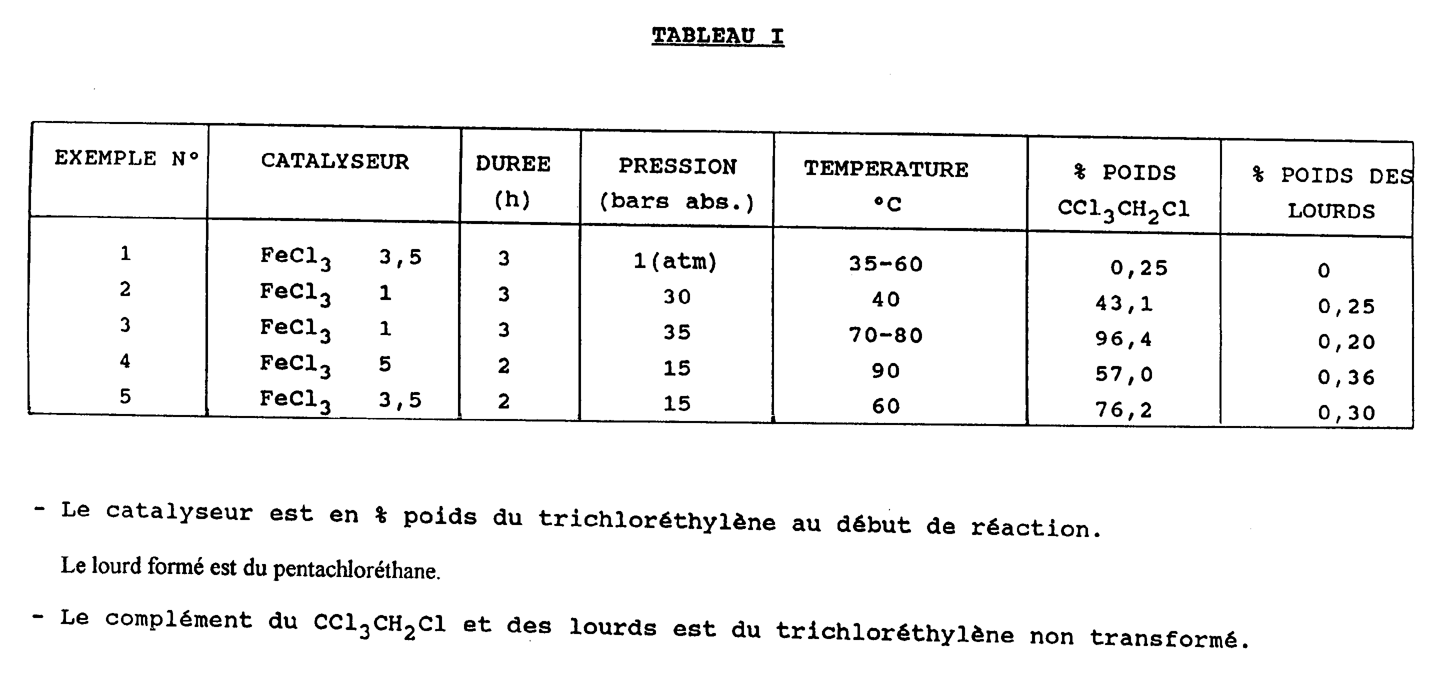

## Revendications

1. Procédé de synthèse du 1,1,1,2-tétrachloréthane par hydrochloration du trichloréthylène en présence de chlorure ferrique caractérisé en ce qu'on opère à une pression supérieure à la pression atmosphérique.

2. Procédé selon la revendication 1 caractérisé en ce que la pression est supérieure à 5 bars absolus et de préférence comprise entre 10 et 40 bars absolus.

3. Procédé selon la revendication 1 ou 2, caractérisé en ce que la quantité de chlorure ferrique est comprise entre 0,1 et 20 % et de préférence 1 et 5 % en poids du trichloréthylène.

4. Procédé selon l'une des revendications 1 à 3, caractérisé en ce que la température est comprise entre 10 et 160°C.

## Claims

1. Process for the synthesis of 1,1,1,2-tetrachloroethane by hydrochlorination of trichloroethylene in the presence of ferric chloride, characterized in that the process is carried out at a pressure greater than atmospheric pressure.

2. Process according to Claim 1, characterized in that the pressure is greater than 5 bars absolute and is preferably between 10 and 40 bars absolute.

3. Process according to Claim 1 or 2, characterized in that the amount of ferric chloride is between 0.1 and 20 % and preferably between 1 and 5 % by weight relative to the weight of the trichloroethylene.

4. Process according to one of Claims 1 to 3, characterized in that the temperature is between 10 and 160°C.

## Patentansprüche

1. Verfahren zur Herstellung von 1,1,1,2-Tetrachlorethan durch Hydrochlorierung von Trichlorethylen in Gegenwart von Eisen-(III)-chlorid, dadurch gekennzeichnet, daß man bei einem Druck über dem atmosphärischen Druck arbeitet.

2. Verfahren nach Anspruch 1, dadurch gekennzeichnet, daß der Druck größer als 5 bar ist und vorzugsweise zwischen 10 und 40 bar liegt.

3. Verfahren nach Anspruch 1 oder 2, dadurch gekennzeichnet, daß die Menge an Eisen-(III)-chlorid zwischen 0,1 und 20, vorzugsweise zwischen 1 und 5 Gew.%, bezogen auf Trichlorethylen, liegt.

4. Verfahren nach einem der Ansprüche 1 bis- 3, dadurch gekennzeichnet, daß die Temperatur zwischen 10 und 160 °C liegt.
